# EUROPEAN PATENT APPLICATION

(11) **EP 4 624 927 A1**
(43) Date of publication of application: **01.10.2025**
(21) Application number: 23893681.9
(22) Date of filing: 14.11.2023
(51) Int. Cl.: G01N 33/531, G01N 33/543

(54) **PROTEIN-COATED MAGNETIC BEAD, AND PREPARATION METHOD THEREFOR, USE THEREOF, AND METHOD FOR DETECTING TARGET ANTIBODY**

(30) Priority: 23.11.2022 CN 202211474564
(71) Applicant: Shenzhen Yhlo Biotech Co., Ltd., Shenzhen, Guangdong 518116 (CN)
(72) Inventor: XU, Jingjing, Shenzhen, Guangdong 518116 (CN); WANG, Gang, Shenzhen, Guangdong 518116 (CN); CHEN, Yongtang, Shenzhen, Guangdong 518116 (CN); QIAN, Chungen, Shenzhen, Guangdong 518116 (CN)
(74) Representative: Leonard, Thomas Charles
(86) International application number: PCT/CN2023/131557
(87) International publication number: WO 2024/109591

(57) **Abstract**

A protein-coated magnetic bead comprising a magnetic base sphere unit; a protein or polypeptide unit; a polyethylene glycol chain, wherein one end of the polyethylene glycol chain is connected to the magnetic base sphere unit by means of a thioether bond and the other end is connected to the end of the protein or polypeptide unit by means of a carbon-nitrogen double bond. Since in the protein-coated magnetic bead, the polyethylene glycol chain is located away from an active site of the protein or polypeptide unit, when an antibody or antigen corresponding to the protein or polypeptide unit is detected, the problem of decreased detection rate caused by the covering of the active site of the protein or polypeptide unit can be avoided and the detection rate is increased. **In** addition, since the polyethylene glycol chain has affinity and hydrophilicity, and good safety and biocompatibility, the structural stability of the protein or polypeptide unit can be improved, and the conformational change of the protein or polypeptide unit in a coupling process can be avoided.

## Description

### TECHNICAL FIELD

The present application relates to the technical field, in particular to a protein-coated magnetic bead, a preparation method thereof, an application thereof, and a method for detecting a target antibody.

### BACKGROUND

Immunoassay and immuno-diagnosis in chemiluminescence platforms are performed by methods including binding antigens or antibodies to the surface of nano-magnetic beads through physical adsorption or chemical crosslinking, while the antigens or antibodies maintain their immunological activity. During the detection, a sample to be tested is first immunologically reacted with the antigen or antibody on the surface of the magnetic bead, then is bound with a tracer-labeled antigen or antibody to form a complex, and is quantitatively or qualitatively evaluated based on luminescence through different steps.

In biomedicine, polyethylene glycol (PEG) is widely used to covalently couple with proteins or peptides to change their biochemical properties, due to its affinity and hydrophilicity, as well as good safety and biocompatibility. However, during the detection, there are problems such as suppression of positive signal values, and signal-to-noise ratio and detection rate failing to meet performance requirements.

### SUMMARY

Based on this, there is a need to provide a protein-coated magnetic bead, a preparation method thereof, an application thereof, and a method for detecting a target antibody, which can improve the signal-to-noise ratio and detection rate during detection.

A first aspect of the present application provides a protein-coated magnetic bead, which includes a magnetic core bead unit, a protein or polypeptide unit, and a polyethylene glycol chain. One end of the polyethylene glycol chain is connected to the magnetic core bead unit through a thioether bond, and another end of the polyethylene glycol chain is connected to an end of the protein or polypeptide unit through a carbon-nitrogen double bond.

In some embodiments, the polyethylene glycol chain includes an even number of carbon atoms.

Optionally, the polyethylene glycol chain includes 2 to 24 carbon atoms.

In some embodiments, raw materials for preparing the protein-coated magnetic bead include a magnetic core bead with a surface conjugated with an amino group, a protein or polypeptide with an α-amino group at an end thereof, and polyethylene glycol aldehyde with a thiol group and an aldehyde group at two ends thereof respectively.

In some embodiments, the thioether bond is generated by reaction between the thiol group at one end of the polyethylene glycol aldehyde and the amino group on the surface of the magnetic core bead; and the carbon-nitrogen double bond is generated by reaction between the aldehyde group at another end of the polyethylene glycol aldehyde and the α-amino group at the end of the protein or polypeptide.

In some embodiments, the protein or polypeptide includes one or more of gp210 antigen, sp100 antigen, thyroglobulin, or histone.

A second aspect of the present application provides a method for preparing the protein-coated magnetic bead, including:
preforming a first reaction between the polyethylene glycol aldehyde with a thiol group and an aldehyde group at two ends thereof respectively and the protein or polypeptide with an α-amino group at an end thereof under action of a reducing agent to prepare an intermediate; and
preforming a second reaction between the intermediate and magnetic core beads with a surface conjugated with an amino group under action of a crosslinking agent to obtain protein-coated magnetic beads.

In some embodiments, the intermediate is prepared under at least one of following conditions:
(1) a molar ratio of the protein or polypeptide, the polyethylene glycol aldehyde, and the reducing agent is 1:(5 to 40):200;
(2) the reducing agent includes one or more of sodium borohydride, sodium borohydride acetate, or sodium cyanoborohydride;
(3) a solvent configured to dissolve the polyethylene glycol aldehyde in the first reaction includes one or more of water, N,N-dimethylformamide, or dimethyl sulfoxide;
(4) a pH value of the first reaction ranges from 4 to 5;
(5) a temperature of the first reaction is 4°C, and a reaction time of the first reaction ranges from 16 h to 18 h; or a temperature of the first reaction is 25°C, and a reaction time of the first reaction ranges from 6 h to 8 h.

In some embodiments, the second reaction is performed under at least one of following conditions:
(1) the crosslinking agent includes either or both of succinimidyl 4-(N-maleimidomethyl)cyclohexane-1-carboxylate, and 4-(N-Maleimidomethyl)cyclohexane-1-carboxylic acid 3-sulfo-N-hydroxysuccinimide ester sodium salt;
(2) a mass ratio of the magnetic core beads to the crosslinking agent is 1:(0.05 to 0.2);
(3) a mass ratio of the protein or polypeptide contained in the intermediate to the magnetic core beads is (5 to 40): 1000;
(4) the second reaction specifically includes:
   mixing the dissolved crosslinking agent with the magnetic core beads to obtain a mixture, and activating the mixture;
   mixing the activated mixture with the intermediate to couple the magnetic core beads with the intermediate; and
   blocking the coupled product.

A third aspect of the present application provides use of the protein-coated magnetic bead described above in the preparation of a diagnostic reagent.

A fourth aspect of the present application provides a diagnostic reagent, including the protein-coated magnetic bead described above or the protein-coated magnetic bead prepared by the method described above.

A fifth aspect of the present application provides a method for detecting a target antibody, including:
preforming a first reaction between polyethylene glycol aldehyde with a thiol group and an aldehyde group at two ends thereof respectively and a target antigen under action of a reducing agent to prepare an intermediate; wherein the target antigen is an antigen specific to the target antibody, and the target antigen includes an α-amino group at an end thereof;
preforming a second reaction between the intermediate and magnetic core beads with a surface conjugated with an amino group under action of a crosslinking agent to obtain antigen-coated magnetic beads; and
using the antigen-coated magnetic beads to detect the target antibody in a sample solution to be tested.

In some embodiments, the intermediate is prepared under at least one of following conditions:
(1) a molar ratio of the target antigen, the polyethylene glycol aldehyde, and the reducing agent is 1:(5 to 40):200;
(2) the reducing agent includes one or more of sodium borohydride, sodium borohydride acetate , or sodium cyanoborohydride;
(3) a solvent configured to dissolve the polyethylene glycol aldehyde in the first reaction includes one or more of water, N,N-dimethylformamide, or dimethyl sulfoxide;
(4) a pH value of the first reaction ranges from 4 to 5;
(5) a temperature of the first reaction is 4°C, and a reaction time of the first reaction ranges from 16 h to 18 h; or a temperature of the first reaction is 25°C, and a reaction time of the first reaction ranges from 6 h to 8 h.

In some embodiments, the second reaction is performed under at least one of following conditions:
(1) the crosslinking agent includes either or both of succinimidyl 4-(N-maleimidomethyl)cyclohexane-1-carboxylate, and 4-(N-Maleimidomethyl)cyclohexane-1-carboxylic acid 3-sulfo-N-hydroxysuccinimide ester sodium salt;
(2) a mass ratio of the magnetic core beads to the crosslinking agent is 1:(0.05 to 0.2);
(3) a mass ratio of the target antigen contained in the intermediate to the magnetic core beads is (5 to 40): 1000;
(4) the second reaction specifically includes:
   mixing the dissolved crosslinking agent with the magnetic core beads to obtain a mixture, and activating the mixture;
   mixing the activated mixture with the intermediate to couple the magnetic core beads with the intermediate; and
   blocking the coupled product.

According to the protein-coated magnetic bead, the preparation method thereof, the application thereof, and the method for detecting a target antibody proposed above, one end of the polyethylene glycol chain is connected to an end of the protein or polypeptide unit, thereby uniformly modifying the protein or polypeptide unit. Since the polyethylene glycol chain is located away from an active site of the protein or polypeptide unit, when an antibody or antigen specific to the protein or polypeptide unit is detected, the problem of decreased detection rate caused by covering the active site of the protein or polypeptide unit can be avoided and the detection rate is increased. In addition, since the polyethylene glycol chain has affinity and hydrophilicity, and good safety and biocompatibility, the structural stability of the protein or polypeptide unit can be improved, and the conformational change of the protein or polypeptide unit in a coupling process can be avoided.

### DETAILED DESCRIPTION

The present application will now be described more comprehensively with reference to the related embodiments in order to make the present application easily understood. Preferred embodiments of the present application are provided in the following embodiments. However, the present application can be implemented in many different forms and is not limited to the embodiments described herein. Rather, the purpose of providing these embodiments is to make the disclosure of the present application to be understood more thorough and comprehensive.

Unless otherwise defined, all technical and scientific terms used herein have the same meaning as commonly understood by those skilled in the art to which the present application belongs. The terminology used herein in the description of the present application is merely for the purpose of describing specific embodiments, and is not intended to limit the present application.

In the present application, the technical features described in an open-type manner include both closed technical solutions composed of the listed features, and open-type technical solutions containing the listed features.

In the present application, when referring to a numerical range, unless otherwise specified, the numerical range is deemed to be continuous, and includes the minimum and maximum values of the range as well as each value between such minimum and maximum values. Further, when a range refers to an integer, each integer between the minimum and maximum values of the range is included. In addition, when multiple ranges are provided to describe features or characteristics, the ranges can be merged. In other words, unless otherwise specified, all ranges disclosed herein should be understood to include any or all subranges included therein.

In the present application, when referring to the unit of a data range, if a unit is only recited after the right endpoint, it means that the unit of the left endpoint is the same as that of the right endpoint. For example, 6 to 8 h means that the units of the left endpoint "6" and the right endpoint "8" are both h (hours).

Only some numerical ranges are specifically disclosed herein. However, any lower limit can be combined with any upper limit to form an unspecified range, any lower limit can be combined with other lower limits to form unspecified ranges, and any upper limit can be combined with any other upper limit to form an unspecified range. In addition, each point or single value separately disclosed can be taken as a lower limit or upper limit, and combined with any other point or single value or with any other lower limit or upper limit to form an unspecified range.

In the present application, the temperature parameters allow for both constant temperature treatment and variations within a certain temperature range, unless otherwise specified. The constant temperature treatment allows the temperature to fluctuate within the precision range controlled by the instrument.

In addition, the terms "first" and "second" are used for descriptive purposes only and are not to be construed as indicating or implying relative importance or as implicitly specifying the quantity of the technical features indicated. In the description of the present application, "multiple" means at least two, such as two, three, etc., unless otherwise clearly and specifically defined.

Unless otherwise specifically described, all embodiments and optional embodiments of the present application can be combined with each other to form a new technical solution. Unless otherwise specifically described, all technical features and optional technical features of the present application can be combined with each other to form a new technical solution.

Unless otherwise specifically described, all the steps of the present application can be performed sequentially or randomly, preferably performed sequentially.

In biomedicine, polyethylene glycol (PEG) is widely used to covalently couple with proteins or peptides to change their biochemical properties, due to its affinity and hydrophilicity, as well as good safety and biocompatibility. At present, peptides and proteins are modified by the free condensation reaction between their amino groups and carboxyl groups on the PEG chain to stabilize the protein structure thereof. The modified product is coupled with magnetic beads to obtain coated magnetic beads, which are then tested. During the test, there are problems such as suppression of positive signal values, and signal-to-noise ratio and detection rate failing to meet performance requirements. The technicians have found through analysis that the reason may be that random modification may cause active sites of the protein with abundant free amino groups on its surface to be covered, resulting in a decrease in the detection rate.

For example, ε-NH₂ or α-NH₂ of lysine is generally used as the modification target in common PEG modification methods. Multiple lysines in the protein are modified during the modification, and the resulting product is a mixture of PEGylated modified isomers. Due to an unclear structure and highly random modification, the antibody-antigen binding sites are likely to be covered, resulting in a relatively low proportion of obtained target conjugates. Despite the increased stability of the product, the positive signal value is suppressed, resulting in the signal-to-noise ratio and detection rate failing to meet performance requirements.

In order to solve these problems, the present application provides a protein-coated magnetic bead, which includes a magnetic core bead unit, a protein or polypeptide unit, and a polyethylene glycol chain. One end of the polyethylene glycol chain is connected to the magnetic core bead unit through a thioether bond, and another end of the polyethylene glycol chain is connected to an end of the protein or polypeptide unit through a carbon-nitrogen double bond.

It should be noted that, a protein or polypeptide unit refers to a unit composed of a protein, or a unit composed of a polypeptide, or a unit composed of a protein and a polypeptide.

The protein-coated magnetic bead includes one magnetic core bead. The protein-coated magnetic bead includes one protein or polypeptide unit, or multiple protein or polypeptide units. The protein-coated magnetic bead includes one polyethylene glycol chain, or multiple polyethylene glycol chains. The protein or polypeptide unit can be each coupled to the magnetic core bead unit through one polyethylene glycol chain or through multiple polyethylene glycol chains, which is not specifically limited.

It can be understood that, in the protein-coated magnetic bead, one end of the polyethylene glycol chain is connected to an end of the protein or polypeptide unit, thereby uniformly modifying the protein or polypeptide unit. Since the polyethylene glycol chain is located away from an active site of the protein or polypeptide unit, when an antibody or antigen specific to the protein or polypeptide unit is detected, the problem of decreased detection rate caused by covering the active site of the protein or polypeptide unit can be avoided and the detection rate is increased. In addition, since the polyethylene glycol chain has affinity and hydrophilicity, and good safety and biocompatibility, the structural stability of the protein or polypeptide unit can be improved, and the conformational change of the protein or polypeptide unit in a coupling process can be avoided.

In some embodiments, the polyethylene glycol chain includes an even number of carbon atoms. Optionally, the polyethylene glycol chain includes 2 to 24 carbon atoms, for example, can include 4, 6, 8, 10, 12, 14, 16, 18, 20, 22, 24, etc. carbon atoms, which is not specifically limited. Preferably, the polyethylene glycol chain has a linear structure.

In some embodiments, raw materials for preparing the protein-coated magnetic bead include a magnetic core bead with a surface conjugated with an amino group, a protein or polypeptide with an α-amino group at an end thereof, and polyethylene glycol aldehyde with a thiol group and an aldehyde group at two ends thereof respectively.

It should be noted that, the polyethylene glycol aldehyde has a thiol group at one end thereof, and has an aldehyde group at another end thereof. Preferably, the polyethylene glycol aldehyde has a linear structure.

In the backbone of the protein or polypeptide, the pKa value of α-NH₂ of the terminal amino acid residue is usually less than the pKa value of ε-NH₂ or α-NH₂ of lysine. Therefore, in weak acid conditions, polyethylene glycol aldehyde is more likely to undergo a reductive amination reaction with the α-NH₂ of the terminal amino acid residue. When the protein-coated magnetic bead is prepared by using the protein or polypeptide with an α-amino group at an end thereof, the magnetic core bead with a surface conjugated with an amino group, and polyethylene glycol aldehyde with a thiol group and an aldehyde group at two ends thereof respectively, the aldehyde group at the end of polyethylene glycol aldehyde undergoes the reductive amination reaction with the α-amino group at the end of the protein or polypeptide in a weakly acidic environment to form a carbon-nitrogen double bond, which can achieve the effect of directed modification of PEG at the inactive site of the protein or polypeptide, thereby improving the structural stability of the protein or polypeptide unit, while avoiding the problem of occurrence of decreased detection rate during antigen or antibody detection via the protein-coated magnetic bead.

In some embodiments, the thioether bond is generated by reaction between the thiol group at one end of the polyethylene glycol aldehyde and the amino group on the surface of the magnetic core bead; and the carbon-nitrogen double bond is generated by reaction between the aldehyde group at another end of the polyethylene glycol aldehyde and the α-amino group at the end of the protein or polypeptide.

In some embodiments, the protein or polypeptide includes one or more of gp210 antigen, sp100 antigen, thyroglobulin, or histone.

The present application further provides a method for preparing a protein-coated magnetic bead, including the following steps:

preforming a first reaction between the polyethylene glycol aldehyde with a thiol group and an aldehyde group at two ends thereof respectively and the protein or polypeptide with an α-amino group at an end thereof under action of a reducing agent to prepare an intermediate; and

preforming a second reaction between the intermediate and magnetic core beads with a surface conjugated with an amino group under action of a crosslinking agent to obtain the protein-coated magnetic beads.

In the present application, when preparing the protein-coated magnetic bead, the aldehyde group at one end of polyethylene glycol aldehyde is reductively aminated with the α-amino group at the end of the protein or polypeptide to form a carbon-nitrogen double bond, thereby coupling the two and realizing directed modification of the protein or polypeptide at the inactive site. Then, the thiol group at another end of polyethylene glycol aldehyde undergoes addition reaction with the amino group on the surface of the magnetic core bead to form a stable thioether bond, thereby coupling the magnetic core bead with the polyethylene glycol aldehyde and the protein or polypeptide, and obtaining the protein-coated magnetic bead with stable protein or polypeptide unit structure and a high detection rate.

It should be noted that, the addition reaction between the thiol group and the amino group needs to be carried out under the catalytic action of a crosslinking agent. In the absence of the crosslinking agent, the thiol group cannot react with the amino group. Therefore, when the polyethylene glycol aldehyde reacts with the protein or polypeptide, the thiol group at the end of polyethylene glycol aldehyde does not affect the reductive amination between the aldehyde group and the amino group, so that high-purity and uniform modification of the protein or polypeptide can be achieved, and a purified and uniform modified product is obtained.

It can be understood that, the first reaction is a reductive amination reaction, and the second reaction is an addition reaction.

In some embodiments, when preparing the intermediate, a molar ratio of the protein or polypeptide, polyethylene glycol aldehyde, and the reducing agent is 1:(5 to 40):200; for example, it can be 1:(5 to 35):200, 1:(5 to 30):200, 1:(5 to 25):200, 1:(5 to 20):200, 1:(5 to 15):200, 1:(5 to 10):200, 1:(5 to 9):200, 1:(5 to 8):200, 1:(5 to 7):200, or 1:(5 to 6):200, etc; preferably, it is 1:(5 to 10):200. When the molar ratio of the protein or polypeptide, polyethylene glycol aldehyde, and the reducing agent is within the above range, the protein or polypeptide can be effectively modified with polyethylene glycol aldehyde.

In some embodiments, when preparing the intermediate, the reducing agent includes one or more of sodium borohydride, sodium borohydride acetate, or sodium cyanoborohydride, preferably sodium cyanoborohydride.

In some embodiments, when preparing the intermediate, a solvent configured to dissolve the polyethylene glycol aldehyde in the first reaction includes one or more of water, N,N-dimethylformamide, or dimethyl sulfoxide.

In some embodiments, a pH value of the first reaction ranges from 4 to 5. For example, the pH value can range from 4.1 to 5, 4.2 to 5, 4.3 to 5, 4.4 to 5, 4.5 to 5, 4.6 to 5, 4.7 to 5, 4.8 to 5, 4.9 to 5, etc. Preferably, the pH value is 5. The first reaction is carried out in a weakly acidic environment, so that the aldehyde group at the end of polyethylene glycol aldehyde can selectively undergo the reductive amination reaction with the α-amino group of the terminal amino acid residue of the protein or polypeptide, thereby achieving a directed modification of the protein or polypeptide at the inactive site.

In some embodiments, the first reaction can be carried out at 4°C for 16 h to 18 h, or at 25°C for 6 h to 8 h.

As an example, the intermediate can be prepared according to the following steps. A certain amount of polyethylene glycol aldehyde dry powder is weighed and dissolved in dimethyl sulfoxide to prepare 1 mg/mL of polyethylene glycol aldehyde solution. An acetic acid-sodium acetate buffer with a pH of 5 and a concentration of 40 mmol/L is prepared as a reaction buffer. The protein, the polyethylene glycol aldehyde, and the reducing agent in a specific molar ratio are reacted at 4°C for 16 h to 18 h, or at 25°C for 6 h to 8 h. After completion of the reaction, the intermediate is purified via ultrafiltration. Optionally, the intermediate purified by ultrafiltration can be stored in a 20 mmol/L of phosphate buffer with a pH of 7.4 for standby use.

In some embodiments, the crosslinking agent used in the second reaction includes either or both of succinimidyl 4-(N-maleimidomethyl)cyclohexane-1-carboxylate, and 4-(N-Maleimidomethyl)cyclohexane-1-carboxylic acid 3-sulfo-N-hydroxysuccinimide ester sodium salt, preferably, succinimidyl 4-(N-maleimidomethyl)cyclohexane-1-carboxylate.

In some embodiments, a mass ratio of the magnetic core beads to the crosslinking agent in the second reaction is 1:(0.05 to 0.2); for example, can be 1:(0.05 to 0.18), 1:(0.05 to 0.15), 1:(0.05 to 0.12), 1:(0.05 to 0.1), 1:(0.05 to 0.08), etc., which is not specifically limited. When the mass ratio of the magnetic core beads to the crosslinking agent is within the above range, the crosslinking agent can fully activate the groups on the surfaces of the magnetic core beads.

In some embodiments, in the second reaction, a mass ratio of the protein or polypeptide contained in the intermediate to the magnetic core beads is (5 to 40):1000; for example, can be (5 to 35):1000, (10 to 30):1000, (15 to 25):1000, (15 to 20):1000, (20 to 25):1000, etc., which is not specifically limited. When the mass ratio of the protein or polypeptide contained in the intermediate to the magnetic core beads is within the above range, it can ensure that a sufficient amount of protein or polypeptide is coupled to the magnetic core beads, while the problem of decrease in coupling efficiency caused by increased steric hindrance due to an excessive amount of protein does not occur.

In some embodiments, the second reaction specifically includes: mixing the dissolved crosslinking agent with the magnetic core beads to obtain a mixture, and activating the mixture; mixing the activated mixture with the intermediate to couple the magnetic core beads with the intermediate; and blocking the coupled product.

As an example, the mixture is activated by the following steps. The crosslinking agent is dissolved in dimethyl sulfoxide to prepare 10 mg/mL of crosslinking agent solution. Then, 50 µL of the crosslinking agent solution is added to the magnetic core bead solution according to 1:0.05 of the mass ratio of magnetic core beads to succinimidyl 4-(N-maleimidomethyl)cyclohexane-1-carboxylate, and the mixture is activated in an incubator at a constant temperature of 25°C for 60 mins.

As an example, the magnetic core beads and the intermediate are coupled according to the following steps. The activated mixture of the magnetic core beads and the crosslinking agent is magnetically separated, and the supernatant is discarded to obtain the solid. The solid is washed once with 0.02 mol/L of HEPES buffer having pH of 7.0, and then resuspended with 0.02 mol/L of HEPES buffer having pH of 7.0. The intermediate is added to the suspension solution, mixed uniformly, and placed in an incubator at a constant temperature of 25°C for reaction under mixing for 3 hours.

As an example, blocking the coupled product is performed according to the following steps. The coupled product is magnetically separated, the supernatant is discarded to obtain the solid, 0.15 mol/L of phosphate buffer containing 0.5% BSA with pH of 7.4 is added as a blocking buffer, placed in an incubator at a constant temperature of 25°C, and blocked under mixing for 2 hours.

In some embodiments, the blocking buffer can contain one or more of protein, non-protein, antibody, or serum as a blocking agent, preferably, protein.

In some embodiments, after preparing the intermediate, the method further includes purifying the intermediate. Optionally, the intermediate can be purified by desalting, ultrafiltration, or dialysis, preferably ultrafiltration.

The present application further provides use of the above-mentioned protein-coated magnetic bead in the preparation of a diagnostic reagent.

The present application further provides a diagnostic reagent, including the above-mentioned protein-coated magnetic bead or the protein-coated magnetic bead prepared by the above-mentioned method.

The present application further provides a method for detecting a target antibody, including the following steps:
preforming a first reaction between polyethylene glycol aldehyde with a thiol group and an aldehyde group at two ends thereof respectively and a target antigen under action of a reducing agent to prepare an intermediate; wherein the target antigen is an antigen specific to the target antibody, and the target antigen includes an α-amino group at an end thereof;
preforming a second reaction between the intermediate and the magnetic core beads with a surface conjugated with an amino group under action of a crosslinking agent to obtain the antigen-coated magnetic beads; and
using the antigen-coated magnetic beads to detect the target antibody in a sample solution to be tested.

In the present application, when the target antibody is detected, the aldehyde group at one end of polyethylene glycol aldehyde is reductively aminated with the α-amino group at the end of the target antigen to form a carbon-nitrogen double bond, thereby coupling the two and realizing directed modification of the target antigen at the inactive site. Then, the thiol group at another end of polyethylene glycol aldehyde undergoes addition reaction with the amino group on the surface of the magnetic core bead to form a stable thioether bond, thereby coupling the magnetic core bead with the polyethylene glycol aldehyde and the target antigen, and obtaining the protein-coated magnetic bead with stable target antigen structure and a high detection rate.

It can be understood that, when the target antibody is detected, the target antigen specific to the target antibody is first modified with PEG, and then coupled with the magnetic core bead to prepare the antigen-coated magnetic bead. Since PEG has affinity and hydrophilicity, and good safety and biocompatibility, modifying the target antigen by PEG can stabilize the structure of the target antigen, and the conformational change of the target antigen in a coupling process can be avoided. Meanwhile, the PEG performs site-specific modification to the target antigen at the end of the target antigen, away from the active site of the target antigen, to obtain a relatively uniform modified product, and the problem of decreased detection rate caused by modification can be avoided.

Compared with the cases not modifying the target antigen or modifying the target antigen with other PEG derivatives, the antigen-coated magnetic bead of the present application, when used to detect the target antibody, can effectively improve the detection rate of the target antibody.

In some embodiments, when preparing the intermediate, the molar ratio of the target antigen, polyethylene glycol aldehyde, and the reducing agent is 1:(5 to 40):200, for example, can be 1:(5 to 35):200, 1:(5 to 30):200, 1:(5 to 25):200, 1:(5 to 20):200, 1:(5 to 15):200, 1:(5 to 10):200, 1:(5 to 9):200, 1:(5 to 8):200, 1:(5 to 7):200, 1:(5 to 6):200, etc., preferably, 1:(5 to 10):200.

In some embodiments, when preparing the intermediate, the reducing agent includes one or more of sodium borohydride, sodium borohydride acetate, or sodium cyanoborohydride, preferably, sodium cyanoborohydride.

In some embodiments, when preparing the intermediate, the solvent configured to dissolve the polyethylene glycol aldehyde in the first reaction includes one or more of water, N,N-dimethylformamide, or dimethyl sulfoxide.

In some embodiments, a pH value of the first reaction ranges from 4 to 5; for example, can be 4.1 to 5, 4.2 to 5, 4.3 to 5, 4.4 to 5, 4.5 to 5, 4.6 to 5, 4.7 to 5, 4.8 to 5, 4.9 to 5, etc; preferably, can be 5. The first reaction is carried out in a weakly acidic environment, so that the aldehyde group at the end of polyethylene glycol aldehyde can selectively undergo a reductive amination reaction with the α-amino group at the end of the target antigen, thereby achieving a directed modification of the protein or polypeptide at the inactive site.

In some embodiments, the first reaction can be performed at 4°C for 16 h to 18 h, or at 25°C for 6 h to 8 h.

In some embodiments, the crosslinking agent used in the second reaction includes either or both of succinimidyl 4-(N-maleimidomethyl)cyclohexane-1-carboxylate, and 4-(N-Maleimidomethyl)cyclohexane-1-carboxylic acid 3-sulfo-N-hydroxysuccinimide ester sodium salt; preferably, succinimidyl 4-(N-maleimidomethyl)cyclohexane-1-carboxylate.

In some embodiments, a mass ratio of the magnetic core beads to the crosslinking agent in the second reaction is 1:(0.05 to 0.2); for example, can be 1:(0.05 to 0.18), 1:(0.05 to 0.15), 1:(0.05 to 0.12), 1:(0.05 to 0.1), 1:(0.05 to 0.08), etc., without specific limitation.

In some embodiments, a mass ratio of the target antigen contained in the intermediate to the magnetic core beads in the second reaction is (5 to 40):1000; for example, can be (5 to 35):1000, (10 to 30):1000, (15 to 25):1000, (15 to 20):1000, (20 to 25):1000, etc., without specific limitation.

In some embodiments, the second reaction specifically includes: mixing the dissolved crosslinking agent with the magnetic core beads to obtain a mixture, and activating the mixture; mixing the activated mixture with the intermediate to couple the magnetic core beads with the intermediate; and blocking the coupled product.

The technical solution is described below in conjunction with specific embodiments.

### 1. Preparation of protein-coated magnetic bead

It should be noted that, in the following Examples and Comparative Examples, anti-gp210 antibody is detected as an example, and its target antigen is a 210 kD transmembrane glycoprotein located on the nuclear pore complex. Anti-gp210 antibody functions an auxiliary diagnostic role in the diagnosis of patients with autoimmune hepatitis. If a patient is positive for antinuclear antibody and anti-gp210 antibody, consideration is primarily given to autoimmune hepatitis or primary cholestatic hepatitis in the patient.

In the following Examples and Comparative Examples, HS-PEG-CHO is polyethylene glycol aldehyde with an aldehyde group and a thiol group at both ends thereof respectively; DMSO is dimethyl sulfoxide; PBS buffer is phosphate buffer; SMCC is succinimidyl 4-(N-maleimidomethyl)cyclohexane-1-carboxylate.

### Example 1

### 1. Modification of gp210 antigen with HS-PEG-CHO

HS-PEG-CHO with a relative molecular mass of 1000 was dissolved in DMSO to prepare a 1 mg/mL HS-PEG-CHO solution.

100 µg of gp210 antigen with a relative molecular mass of 210 kDa was added to 300 µL of 40 mmol/L acetic acid-sodium acetate buffer with pH of 5.0, and then 6 µL of 1 mg/mL sodium cyanoborohydride aqueous solution was added to obtain an antigen mixture.

2.38 µL of the HS-PEG-CHO solution was added to the antigen mixture, and uniformly mixed to obtain a reaction system with gp210 antigen, HS-PEG-CHO, and sodium cyanoborohydride in a molar ratio of 1:5:200. The reaction system was reacted at 4°C for 16 h to 18 h.

After the completion of reaction, the HS-PEG-CHO modified gp210 antigen (i.e., an intermediate) was obtained. The HS-PEG-CHO modified gp210 antigen was purified by ultrafiltration, and stored in 20 mmol/L PBS buffer with pH of 7.4.

### 2. Preparation of modified antigen-coated magnetic beads

10 mg of magnetic beads with a surface conjugated with amino groups were washed three times with 0.02 mol/L PBS buffer with pH of 7.4, and then resuspend in 0.4 mL of PBS buffer.

SMCC was dissolved in DMSO to prepare a 10 mg/mL SMCC solution. Then, based on a mass ratio of 1:0.05 of magnetic beads to SMCC, 50 µL of SMCC solution was added to the magnetic beads, and activated in an incubator at 25°C for 60 mins.

The activated magnetic bead solution was subjected to magnetic separation, and the supernatant was discarded. The solid was washed once with 1 mL of 0.02 mol/L HEPES buffer with pH of 7.0, and resuspended in the HEPES buffer. Then, 100 µg of HS-PEG-CHO modified gp210 antigen was added, mixed uniformly, and placed in an incubator at 25°C for reaction under mixing for 3 h.

After the reaction was completed, the reaction product was magnetically separated. 0.5% BSA-containing phosphate buffer with pH of 7.4 and a concentration of 0.15 mol/L was added, and the mixture was mixed and blocked in an incubator at 25°C for 2 h to obtain modified antigen-coated magnetic beads.

After the blocking reaction was completed, the modified antigen-coated magnetic beads were magnetically separated, then fixed to a concentration of 10 mg/mL with 0.5% BSA-containing phosphate buffer, and stored at 4°C until use.

### Example 2

Example 2 is basically the same as Example 1, only with the difference in the molar ratio of gp210 antigen, HS-PEG-CHO, and sodium cyanoborohydride. The details are as follows.

### 1. Modification of gp210 antigen with HS-PEG-CHO

HS-PEG-CHO with a relative molecular mass of 1000 was dissolved in DMSO to prepare a 1 mg/mL HS-PEG-CHO solution.

100 µg of gp210 antigen with a relative molecular mass of 210 kDa was added to 300 µL of 0.04 mmol/L acetic acid-sodium acetate buffer with pH of 5.0, and then 6 µL of 1 mg/mL sodium cyanoborohydride aqueous solution was added to obtain an antigen mixture.

4.76 µL of the HS-PEG-CHO solution was added to the antigen mixture, and uniformly mixed to obtain a reaction system with gp210 antigen, HS-PEG-CHO, and sodium cyanoborohydride in a molar ratio of 1: 10:200. The reaction system was reacted at 4°C for 16 h to 18 h.

After the completion of reaction, the HS-PEG-CHO modified gp210 antigen (i.e., an intermediate) was obtained. The HS-PEG-CHO modified gp210 antigen was purified by ultrafiltration, and stored in 20 mmol/L PBS buffer with pH of 7.4.

### 2. The preparation of modified antigen-coated magnetic beads is the same as that in Example 1.

### Example 3

Example 3 is basically the same as Example 1, only with the difference in different reaction temperature and different reaction time for preparing HS-PEG-CHO modified gp210 antigen. The details are as follows.

### 1. Modification of gp210 antigen with HS-PEG-CHO

HS-PEG-CHO with a relative molecular mass of 1000 was dissolved in DMSO to prepare a 1 mg/mL HS-PEG-CHO solution.

100 µg of gp210 antigen with a relative molecular mass of 210 kDa was added to 300 µL of 0.04 mmol/L acetic acid-sodium acetate buffer with pH of 5.0, and then 6 µL of 1 mg/mL sodium cyanoborohydride aqueous solution was added to obtain an antigen mixture.

2.38 µL of the HS-PEG-CHO solution was added to the antigen mixture, and uniformly mixed to obtain a reaction system with gp210 antigen, HS-PEG-CHO, and sodium cyanoborohydride in a molar ratio of 1:5:200. The reaction system was reacted at 25°C for 6 h to 8 h.

After the completion of reaction, the HS-PEG-CHO modified gp210 antigen (i.e., an intermediate) was obtained. The HS-PEG-CHO modified gp210 antigen was purified by ultrafiltration, and stored in 20 mmol/L PBS buffer with pH of 7.4.

### 2. The preparation of modified antigen-coated magnetic beads is the same as that in Example 1.

### Comparative Example 1

Comparative Example 1 differs from Example 1 in that a different PEG derivative is used to modify the gp210 antigen. The details are as follows.

### 1. Modification of gp210 antigen with HS-PEG-CHO

H₃C-PEG-NHS ester with a relative molecular mass of 333.3 was dissolved in DMSO to prepare a 1 mg/mL H₃C-PEG-NHS ester solution.

100 µg of gp210 antigen was added to 300 µL of 0.1 mmol/L phosphate buffer with pH of 7.0.

1.6 µL of H₃C-PEG-NHS ester solution was added to the antigen mixture, and uniformly mixed to obtain a reaction system with gp210 antigen and H₃C-PEG-NHS ester in a molar ratio of 1:10. The reaction system was reacted at 25°C for 30 mins.

After the completion of reaction, H₃C-PEG-NHS ester-modified gp210 antigen (i.e., an intermediate) was obtained. The H₃C-PEG-NHS ester -modified gp210 antigen was purified by ultrafiltration, and stored in 20 mmol/L PBS buffer with pH of 7.4.

### 2. The preparation of modified antigen-coated magnetic beads is the same as that in Example 1.

### Comparative Example 2

Comparative Example 2 differs from Example 1 in that the gp210 antigen was not modified, and was directly coupled to the magnetic beads. The details are as follows.

10 mg of magnetic beads with a surface conjugated with carboxyl groups was washed three times with 0.015 mol/L MES buffer with pH of 6.0, and then resuspended in 0.4 mL of MES buffer.

Based on 1:0.1:0.1 of the mass ratio of magnetic beads, carbodiimide, and N-hydroxysuccinimide, carbodiimide and N-hydroxysuccinimide were added to the magnetic bead solution, and reacted at room temperature of 25°C for 30 mins.

The mixture was subjected to magnetic separation, and the supernatant was discarded. The solid was washed with 0.015 mol/L MES buffer with pH of 6.0, and resuspended in the MES buffer to obtain a 10 mg/mL activated magnetic bead solution.

100 µg of gp210 antigen with a relative molecular mass of 210 kDa was added to the activated magnetic bead solution, uniformly mixed, and placed in an incubator at 25°C for reaction under mixing for 3 h.

After the reaction was completed, the reaction product was magnetically separated. 0.5% BSA-containing phosphate buffer solution with pH of 7.4 and a concentration of 0.15 mol/L was added, and the mixture was mixed and blocked in an incubator at 25°C for 2 h.

After the blocking reaction was completed, the antigen-coated magnetic beads were magnetically separated, then fixed to a concentration of 10 mg/mL with 0.5% BSA-containing phosphate buffer with pH of 7.4 and a concentration of 0.15 mol/L, and stored at 4°C until use.

### II. Anti-gp210 antibody test

### 1. Six gradient positive samples were collected to test the coated magnetic beads in Examples 1 to 3 and Comparative Example 2.

The coated magnetic beads prepared in Examples and Comparative Example 2 were each diluted to 0.1 mg/mL. 50 µL of the coated magnetic bead diluent was taken, 100 µL of the sample solution to be tested was added, incubated at 37°C for 10 mins, and then magnetically separated to obtain a first reactant. The first reactant was washed twice with PBS buffer with pH of 7.4. 100 µL of 200 ng/mL acridinium ester-labeled mouse anti-human IgG antibody was added. The mixture was incubated at 37°C for 10 mins, and then magnetically separated to obtain a second reactant. The second reactant was washed twice with PBS buffer with pH of 7.4. 200 µL of luminescent solution was added, and a luminescent signal value was measured by using a chemiluminescence analyzer. The test was performed in duplicate. An average value and deviation of the luminescent signal value were calculated. The results are shown in Table 1.

**Table 1**

| Sample No. | Example 1 | | Example 2 | | Example 3 | | Comparative Example 2 | |
|---|---|---|---|---|---|---|---|---|
| | Signal value | Average | Signal value | Average | Signal value | Average | Signal value | Average |
| 1 | 751369 | 760276 | 864074 | 862780 | 1036889 | 1031028 | 563527 | 567515 |
| | 769183 | | 861485 | | 1025167 | | 571503 | |
| 2 | 356241 | 362713 | 403265 | 410776 | 479079 | 488211 | 278224 | 285125 |
| | 369185 | | 418287 | | 497343 | | 292025 | |
| 3 | 180457 | 179392 | 197059 | 197947 | 234303 | 234663 | 146531 | 145042 |
| | 178327 | | 198835 | | 235023 | | 143553 | |
| 4 | 92629 | 91118 | 101336 | 100623 | 121198 | 120246 | 71139 | 70381 |
| | 89606 | | 99911 | | 119293 | | 69624 | |
| 5 | 33989 | 34271 | 35485 | 35813 | 42298 | 42725 | 27939 | 28291 |
| | 34552 | | 36141 | | 43153 | | 28644 | |
| 6 | 17642 | 17300 | 17960 | 17645 | 21300 | 20910 | 15031 | 14587 |
| | 16958 | | 17331 | | 20520 | | 14143 | |

Through the comparison of results of Examples 1 to 3 and Comparative Example 2 in Table 1, it can be seen that the signal value of the analyte tested with the present site-specifically PEGylated antigen-coated magnetic beads is generally higher than the signal value of the analyte tested with magnetic beads directly coupled with antigen.

According to the results of Example 1 and Example 2, it can be seen that increasing the molar ratio of gp210 antigen to polyethylene glycol aldehyde from 1:5 to 1:10 can further improve the modification efficiency of gp210 antigen by PEG, so that the tested signal value is improved accordingly. According to the results of Example 1 and Example 3, it can be seen that, during preparing HS-PEG-CHO modified gp210 antigen, increasing the reaction temperature from 4°C to 25°C can further improve the modification efficiency of gp210 antigen by PEG, so that the tested signal value is improved accordingly. These results show that when antigens are site-specifically PEGylated, different treatment conditions can affect the modification effect and the tested signal value.

2. The coated magnetic beads of Example 1, Comparative Example 1, and Comparative Example 2 were respectively diluted to a working concentration of 0.10 mg/mL. Eight positive gradient samples and 100 random samples were collected to test the specificity and sensitivity of the coated magnetic beads of Example 1, Comparative Example 1, and Comparative Example 2 in anti-gp210 antibody detection. The specific process was the same as the above process. The test results of specificity are shown in Table 2. The test results of sensitivity are shown in Table 3.

**Table 2**

| Item | Example 1 | Comparative Example 1 | Comparative Example 2 |
|---|---|---|---|
| Specificity | 98.0% | 93.0% | 95.0% |

**Table 3**

| Sample No. | Example 1 | | Comparative Example 1 | | Comparative Example 2 | |
|---|---|---|---|---|---|---|
| | Signal value | Average | Signal value | Average | Signal value | Average |
| 1 | 695581 | 689522 | 492716 | 488920 | 558046 | 553712 |
| | 683462 | | 485123 | | 549377 | |
| 2 | 335716 | 332332 | 238467 | 243315 | 253461 | 256713 |
| | 328947 | | 248163 | | 259964 | |
| 3 | 158947 | 162816 | 116843 | 123141 | 136421 | 133093 |
| | 166685 | | 129438 | | 129764 | |
| 4 | 81144 | 80745 | 58387 | 59206 | 67980 | 68217 |
| | 80345 | | 60024 | | 68453 | |
| 5 | 39237 | 39027 | 23914 | 24051 | 32547 | 31330 |
| | 38816 | | 24187 | | 30113 | |
| 6 | 19186 | 19370 | 12284 | 12596 | 14821 | 14747 |
| | 19553 | | 12907 | | 14673 | |
| 7 | 9547 | 9903 | 4571 | 4539 | 6150 | 6018 |
| | 10258 | | 4506 | | 5885 | |
| 8 | 947 | 958 | 892 | 909 | 952 | 940 |
| | 968 | | 925 | | 928 | |

It can be seen from the results in Table 2 and Table 3 that, the specificity and sensitivity tested with the present site-specifically PEGylated antigen-coated magnetic beads are significantly better than not only the specificity and sensitivity tested with unmodified antigen-coated magnetic bead, but also the specificity and sensitivity tested with randomly modified antigen-coated magnetic bead.

3. Five positive samples and five random samples were collected to test the stability of the coated magnetic beads of Example 1, Comparative Example 1, and Comparative Example 2 in gp210 antigen detection. The specific process was the same as the above process. The test results are shown in Table 4.

**Table 4**

| Samp le No. | Example 1 | | | Comparative Example 1 | | | Comparative Example 2 | | |
|---|---|---|---|---|---|---|---|---|---|
| | Accelera ted at 37°C, Day 0 | Accelera ted at 37°C, Day 7 | Deviati on | Accelera ted at 37°C, Day 0 | Accelera ted at 37°C, Day 7 | Deviati on | Accelera ted at 37°C, Day 0 | Accelera ted at 37°C, Day 7 | Deviati on |
| 1 | 45319 | 35719 | -19.21 % | 30250 | 25176 | -18.35 % | 33989 | 15983 | - 53.04 % |
| | 46503 | 38461 | | 31097 | 24913 | | 34552 | 16206 | |
| 2 | 118603 | 94162 | -17.49 % | 84292 | 64813 | -18.49 % | 92629 | 50044 | -42.82 % |
| | 113282 | 97164 | | 78853 | 68172 | | 89606 | 54160 | |
| 3 | 222238 | 176482 | -19.55 % | 166020 | 134971 | -19.28 % | 180457 | 118371 | - 35.97 |
| | 221524 | 180546 | | 162278 | 130024 | | 178327 | 111352 | |
| 4 | 210490 | 164852 | -18.51 % | 148724 | 115403 | -18.01 % | 161656 | 98368 | -38.01 % |
| | 208389 | 176483 | | 144107 | 124675 | | 161918 | 102209 | |
| 5 | 110061 | 84713 | -17.73 % | 81423 | 66547 | -17.63 % | 90470 | 46266 | -45.82 % |
| | 101993 | 89741 | | 76942 | 63894 | | 84552 | 48568 | |
| 6 | 1716 | 1504 | - 18_{.}11 % | 1287 | 1054 | - 19.74 % | 1462 | 785 | - 46.02 % |
| | 1859 | 1423 | | 1426 | 1123 | | 1550 | 841 | |
| 7 | 1257 | 989 | - 17.03 % | 834 | 719 | - 16.09 % | 917 | 561 | - 47.98 % |
| | 1168 | 1023 | | 953 | 781 | | 1036 | 455 | |
| 8 | 1584 | 1187 | - 20.93 % | 1139 | 995 | - 18.63 % | 1280 | 673 | - 47.00 % |
| | 1613 | 1341 | | 1207 | 914 | | 1341 | 716 | |
| 9 | 1357 | 1191 | - 17.23 % | 968 | 746 | - 21.85 % | 1064 | 611 | - 44.72 % |
| | 1405 | 1095 | | 988 | 783 | | 1123 | 598 | |
| 10 | 1198 | 943 | - 18.37 % | 878 | 715 | - 19.17 % | 987 | 513 | - 51.00 % |
| | 1127 | 955 | | 958 | 769 | | 1064 | 492 | |

It can be seen from the results in Table 4 that, after 7 days of accelerated testing, the signal values in Example 1 and Comparative Example 1 are respectively decreased by 18.4% and 18.7% in average, while the signal value in Comparative Example 2 is decreased by 45% in average, which indicates that the present site-specifically PEGylated antigen exhibits significant improvement in accelerated stability.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the patent protection of the present application shall be defined by the appended claims.

## Claims

1. A protein-coated magnetic bead, **characterized by** comprising:
a magnetic core bead unit;
a protein or polypeptide unit;
a polyethylene glycol chain, wherein one end of the polyethylene glycol chain is connected to the magnetic core bead unit through a thioether bond, and another end of the polyethylene glycol chain is connected to an end of the protein or polypeptide unit through a carbon-nitrogen double bond.

2. The protein-coated magnetic bead according to claim 1, wherein the polyethylene glycol chain comprises an even number of carbon atoms;
optionally, the polyethylene glycol chain comprises 2 to 24 carbon atoms.

3. The protein-coated magnetic bead according to claim 1 or 2, wherein raw materials for preparing the protein-coated magnetic bead comprise a magnetic core bead with a surface conjugated with an amino group, a protein or polypeptide with an α-amino group at an end thereof, and polyethylene glycol aldehyde with a thiol group and an aldehyde group at two ends thereof respectively;
the thioether bond is generated by reaction between the thiol group at one end of the polyethylene glycol aldehyde and the amino group on the surface of the magnetic core bead; and the carbon-nitrogen double bond is generated by reaction between the aldehyde group at another end of the polyethylene glycol aldehyde and the α-amino group at the end of the protein or polypeptide.

4. The protein-coated magnetic bead according to claim 3, wherein the protein or polypeptide comprises one or more of gp210 antigen, sp100 antigen, thyroglobulin, or histone.

5. A method for preparing the protein-coated magnetic bead according to any one of claims 1 to 4, comprising:
preforming a first reaction between the polyethylene glycol aldehyde with a thiol group and an aldehyde group at two ends thereof respectively and the protein or polypeptide with an α-amino group at an end thereof under action of a reducing agent to prepare an intermediate; and
preforming a second reaction between the intermediate and magnetic core beads with a surface conjugated with an amino group under action of a crosslinking agent to obtain protein-coated magnetic beads.

6. The method for preparing the protein-coated magnetic bead according to claim 5, wherein:
the intermediate is prepared under at least one of following conditions:
(1) a molar ratio of the protein or polypeptide, the polyethylene glycol aldehyde, and the reducing agent is 1:(5 to 40):200;
(2) the reducing agent comprises one or more of sodium borohydride, sodium borohydride acetate, or sodium cyanoborohydride;
(3) a solvent configured to dissolve the polyethylene glycol aldehyde in the first reaction comprises one or more of water, N,N-dimethylformamide, or dimethyl sulfoxide;
(4) a pH value of the first reaction ranges from 4 to 5;
(5) a temperature of the first reaction is 4°C, and a reaction time of the first reaction ranges from 16 h to 18 h; or a temperature of the first reaction is 25°C, and a reaction time of the first reaction ranges from 6 h to 8 h,
the second reaction is performed under at least one of following conditions:
(1) the crosslinking agent comprises either or both of succinimidyl 4-(N-maleimidomethyl)cyclohexane-1-carboxylate, and 4-(N-maleimidomethyl)cyclohexane-1-carboxylic acid 3-sulfo-N-hydroxysuccinimide ester sodium salt;
(2) a mass ratio of the magnetic core beads to the crosslinking agent is 1:(0.05 to 0.2);
(3) a mass ratio of the protein or polypeptide contained in the intermediate to the magnetic core beads is (5 to 40): 1000;
(4) the second reaction specifically comprises:
mixing the dissolved crosslinking agent with the magnetic core beads to obtain a mixture, and activating the mixture;
mixing the activated mixture with the intermediate to couple the magnetic core beads with the intermediate; and
blocking the coupled product.

7. Use of the protein-coated magnetic bead according to any one of claims 1 to 4 in the preparation of a diagnostic reagent.

8. A diagnostic reagent, comprising the protein-coated magnetic bead according to any one of claims 1 to 4 or the protein-coated magnetic bead prepared by the method according to claim 5 or 6.

9. A method for detecting a target antibody, comprising:
preforming a first reaction between polyethylene glycol aldehyde with a thiol group and an aldehyde group at two ends thereof respectively and a target antigen under action of a reducing agent to prepare an intermediate; wherein the target antigen is an antigen specific to the target antibody, and the target antigen comprises an α-amino group at an end thereof;
preforming a second reaction between the intermediate and magnetic core beads with a surface conjugated with an amino group under action of a crosslinking agent to obtain antigen-coated magnetic beads; and
using the antigen-coated magnetic beads to detect the target antibody in a sample solution to be tested.

10. The method for detecting the target antibody according to claim 9, wherein the intermediate is prepared under at least one of following conditions:
(1) a molar ratio of the target antigen, the polyethylene glycol aldehyde, and the reducing agent is 1:(5 to 40):200;
(2) the reducing agent comprises one or more of sodium borohydride, sodium borohydride acetate, or sodium cyanoborohydride;
(3) a solvent configured to dissolve the polyethylene glycol aldehyde in the first reaction comprises one or more of water, N,N-dimethylformamide, or dimethyl sulfoxide;
(4) a pH value of the first reaction ranges from 4 to 5;
(5) a temperature of the first reaction is 4°C, and a reaction time of the first reaction ranges from 16 h to 18 h; or a temperature of the first reaction is 25°C, and a reaction time of the first reaction ranges from 6 h to 8 h,
the second reaction is performed under at least one of following conditions:
(1) the crosslinking agent comprises either or both of succinimidyl 4-(N-maleimidomethyl)cyclohexane-1-carboxylate, and 4-(N-Maleimidomethyl)cyclohexane-1-carboxylic acid 3-sulfo-N-hydroxysuccinimide ester sodium salt;
(2) a mass ratio of the magnetic core beads to the crosslinking agent is 1:(0.05 to 0.2);
(3) a mass ratio of the target antigen contained in the intermediate to the magnetic core beads is (5 to 40): 1000;
(4) the second reaction specifically comprises:
mixing the dissolved crosslinking agent with the magnetic core beads to obtain a mixture, and activating the mixture;
mixing the activated mixture with the intermediate to couple the magnetic core beads with the intermediate; and
blocking the coupled product.
